(19) Europäisches Patentamt
European Patent Office
Office européen des brevets

(11) **EP 3 105 350 B1**

(12) **EUROPEAN PATENT SPECIFICATION**

(45) Date of publication and mention
of the grant of the patent:
**10.04.2019 Bulletin 2019/15**

(21) Application number: **15712187.2**

(22) Date of filing: **12.02.2015**

(51) Int Cl.:
**C12Q 1/6883** (2018.01)

(86) International application number:
**PCT/IB2015/000141**

(87) International publication number:
**WO 2015/121737 (20.08.2015 Gazette 2015/33)**

(54) **TRANSCRIPTOMIC BIOMARKERS, METHOD FOR DETERMINATION THEREOF AND USE OF TRANSCRIPTOMIC BIOMARKERS FOR INDIVIDUAL RISK ASSESSMENT OF DEVELOPING POST-INFARCTION HEART FAILURE**

TRANSKRIPTOMISCHE BIOMARKER, VERFAHREN ZUR BESTIMMUNG DAVON UND VERWENDUNG VON TRANSKRIPTOMISCHEN BIOMARKERN ZUR INDIVIDUELLEN RISIKOBEURTEILUNG DER ENTWICKLUNG EINER HERZINSUFFIZIENZ NACH EINEM INFARKT

BIOMARQUEURS TRANSCRIPTOMIQUES, MÉTHODE POUR LEUR DÉTERMINATION ET UTILISATION DE BIOMARQUEURS TRANSCRIPTOMIQUES POUR L'ÉVALUATION DU RISQUE INDIVIDUEL DE DÉVELOPPER UNE INSUFFISANCE CARDIAQUE SUITE À UN INFARCTUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2014 PL 40716314**

(43) Date of publication of application:
**21.12.2016 Bulletin 2016/51**

(73) Proprietors:
- **Instytut Biochemii I Biofizyki Polskiej Akademii Nauk
  02-106 Warszawa (PL)**
- **Warszawski Uniwersytet Medyczny
  02-091 Warszawa (PL)**
- **Uniwersytet Medyczny W Bialymstoku
  15-089 Bialystok (PL)**

(72) Inventors:
- **BURZYNSKA, Beata
  PL-01-457 Warszawa (PL)**
- **GÓRA, Monika
  Warszawa (PL)**
- **MACIEJAK, Agata
  PL-05-600 Grójec (PL)**
- **TULACZ, Dorota
  PL-96-100 Skierniewice (PL)**
- **KILISZEK, Marek
  PL-02-784 Warszawa (PL)**

- **MICHALAK, Marcin
  PL-01-100 Warszawa (PL)**
- **OPOLSKI, Grzegorz
  PL-05-815 Michalowice (PL)**
- **MATLAK, Krzysztof
  PL-05-502 Piaseczno (PL)**
- **DOBRZYCKI, Slawomir
  PL-15-692 Bialystok (PL)**

(74) Representative: **Swiecicki, Krzysztof
Kancelaria Patentowa
Dr inz. Zbigniew Kaminski i Syn
Al. Jerozolimskie 101/18
02-011 Warszawa (PL)**

(56) References cited:
**WO-A1-2013/148316 US-A1- 2010 015 651
US-A1- 2011 294 683**

- **R L Maas ET AL: "A human gene homologous to the formin gene residing at the murine limb deformity locus: chromosomal location and RFLPs", American journal of human genetics, 1 April 1991 (1991-04-01), pages 687-695, XP055196192, UNITED STATES Retrieved from the Internet:
  URL:http://www.ncbi.nlm.nih.gov/pubmed/1673046 [retrieved on 2015]**

**(Cont. next page)**

- Affymetrix: "GeneChip® Gene 1.0 ST Array Design", , 2007, pages 1-8, XP002740974, Retrieved from the Internet: URL:http://static1.1.sqspcdn.com/static/f/1438485/21486042/1357241021290/gene_1_0_st_technote.pdf?token=edawrltG7G51AudOqY1oi0 lxgAs%3D [retrieved on 2015-06-16]
- MAREK KILISZEK ET AL: "Altered Gene Expression Pattern in Peripheral Blood Mononuclear Cells in Patients with Acute Myocardial Infarction", PLOS ONE, vol. 7, no. 11, 21 November 2012 (2012-11-21), page e50054, XP055213701, DOI: 10.1371/journal.pone.0050054
- DATABASE EMBL [Online] 5 January 2012 (2012-01-05), "Gene expression analysis related human gene, SEQ ID NO: 1", XP002744552, Database accession no. AZP92041
- ANNIKA GUSTAFSSON ASTING ET AL: "COX-2 gene expression in colon cancer tissue related to regulating factors and promoter methylation status", BMC CANCER, BIOMED CENTRAL, LONDON, GB, vol. 11, no. 1, 13 June 2011 (2011-06-13), page 238, XP021102353, ISSN: 1471-2407, DOI: 10.1186/1471-2407-11-238
- DOROTA TULACZ ET AL: "Transcriptional profiling of left ventricle and peripheral blood mononuclear cells in a rat model of postinfarction heart failure", BMC MEDICAL GENOMICS, BIOMED CENTRAL LTD, LONDON UK, vol. 6, no. 1, 8 November 2013 (2013-11-08), page 49, XP021166878, ISSN: 1755-8794, DOI: 10.1186/1755-8794-6-49

- Agata Maciejak et al: "Predictive biomarkers in the development of heart failure after acute myocardial infarction", , 9 September 2014 (2014-09-09), XP002740975, Retrieved from the Internet: URL:http://www.actabp.pl/pdf/Supl1_14/Session_6.pdf [retrieved on 2015-06-16]
- AGATA MACIEJAK ET AL: "Gene expression profiling reveals potential prognostic biomarkers associated with the progression of heart failure", GENOME MEDICINE, vol. 4, no. 1, 14 March 2015 (2015-03-14), page 13, XP055195806, DOI: 10.1186/s13073-015-0149-z
- Paul J.R Barton ET AL: "Increased expression of extracellular matrix regulators TIMP1 and MMP1 in deteriorating heart failure", JOURNAL OF HEART AND LUNG TRANSPLANTATION, vol. 22, no. 7, 1 July 2003 (2003-07-01), pages 738-744, XP055389155, US ISSN: 1053-2498, DOI: 10.1016/S1053-2498(02)00557-0

SEQUENCE LISTING

<110> Instytut Biochemii i Biofizyki Polskiej Akademii Nauk
Warszawski Uniwersytet Medyczny

Uniwersytet Medyczny w Białymstoku
<120> Transcriptomic biomarkers, method for determination thereof and
use of transcriptomic biomarkers for individual risk assessment of developing post-infarction heart failure.
<130> 4413p/14
<160> 10
<170> PatentIn version 3.5
<210> 1
<211> 20
<212> DNA
<213> Artificial Sequence
<220>
<223> PCR Primer
<400> 1
ccaagaggat gagctggtta     20
<210> 2
<211> 20

<212> DNA
<213> Artificial Sequence
<220>
<223> PCR Primer
<400> 2
agctcgcgtg atgatctcta          20
<210> 3
<211> 20
<212> DNA
<213> Artificial Sequence
<220>
<223> PCR Primer
<400> 3
gtccggctcc ttctgcttgt          20
<210> 4
<211> 20
<212> DNA
<213> Artificial Sequence
<220>
<223> PCR Primer
<400> 4
gtgtcatatt ccggcgcctc          20
<210> 5
<211> 20
<212> DNA
<213> Artificial Sequence
<220>
<223> PCR Primer
<400> 5
gcttctggca tcctgttgtt          20
<210> 6
<211> 20
<212> DNA
<213> Artificial Sequence
<220>
<223> PCR Primer
<400> 6
aggtggtctg gttgacttct          20
<210> 7
<211> 20
<212> DNA
<213> Artificial Sequence
<220>
<223> PCR Primer
<400> 7
tacgctgaca tccgcaacct          20
<210> 8
<211> 20
<212> DNA
<213> Artificial Sequence
<220>
<223> PCR Primer
<400> 8
aactccgtgc gctccttctt          20
<210> 9
<211> 24
<212> DNA
<213> Artificial Sequence

```
<220>
<223> PCR Primer
<400> 9
tgaccttgat ttattttgca tacc          24
<210> 10
<211> 20
<212> DNA
<213> Artificial Sequence
<220>
<223> PCR Primer
<400> 10
cgagcaagac gttcagtcct          203'
```

## Description

### Field of the invention

The invention relates to medical diagnostics based on molecular biology techniques. More particularly, the invention relates to biomarkers for individual risk assessment of the development of post-infarction heart failure, the method of determining the markers in samples of biological material collected from a patient, use of the biomarkers in the diagnosis (expression level of the gene) that correlate with the development of post-infarction heart failure, and enabling the prevention of heart disease based on a genetic marker and a method for predicting the risk of developing a heart failure for post-infarction patients, as well as a method for optimizing the treatment of cardiac patients with the use of the means of the invention.

### Background of the invention

Biomarkers are molecular, genetic or biochemical factors that are used for the accurate diagnosis of diseases, and the evaluation of which will allow the assessment of pathophysiological conditions. One field in which biomarkers play a particularly important role is the diagnosis, as well as individualization and optimization of treatment, of cardiac patients.

Cardiovascular diseases (including dominant, coronary artery disease, CAD) are the most important cause of mortality in developed countries. Despite continuous advances in diagnosis and treatment, coronary artery disease and heart failure remain a serious medical, social and economic problem (Czech et al., 2013). A myocardial infarction is an event in coronary artery disease often leading to the development of heart failure. The development of heart failure after acute myocardial infarction is directly related to adverse remodelling of the left ventricle. Classical risk factors of heart failure (including infarct size, diabetes) only partially explain why some patients suffer decompensation of the left ventricle, while for others, despite post-infarction damage, it functions quite well. The incidence of heart failure in Poland is approx. 1-3% and continues to grow. One of the causes of the disease includes myocardial infarction (50% of cases) when hypoxia and necrosis of cardiomyocytes occurs, resulting in a reduction in myocardial contractility. Damage to the myocardium can lead to a progressive remodelling of the left ventricle and, as a consequence, to heart failure, which does not occur for all patients after acute coronary events. Hence, it is very important to determine the markers for the easy assessment of patients with progressive dilatation of the left ventricle.

Until today, there have been many different prognostic models created for predicting the risk of death, nonfatal myocardial infarction, or recurrent ischemia in patients with acute coronary syndromes. Besides the basic tools for categorization, which comprise risk factors for coronary heart disease, the heart failure characteristics, ischemic changes in electrocardiogram (ECG), increased levels of markers of myocardial necrosis (troponin, creatine kinase), in the last decade there have been reports on new markers, such as: elevated levels of fibrinogen, C-reactive protein, interleukin-6, B-type natriuretic factor (BNP), and other markers of inflammation or activity of platelets. However, it should be noted that the increase of biochemical biomarkers above reference values may also occur in various disease states, not necessarily associated with myocardial infarction (Iqbal et al., 2012).

Another factor associated with the prognosis of acute coronary syndromes is the remodelling of the left ventricle. Myocardial injury associated with myocardial infarction can lead to a progressive left ventricular remodelling, enlargement of the volume of the left ventricle, a decrease in the ejection fraction and, in consequence, heart failure (Cohn et al., 2000). Modern treatment of acute coronary syndromes - primary coronary angioplasty, Angiotensin-converting enzyme (ACE), beta-blockers - leads to the inhibition of past-infarction heart remodelling. Nevertheless, even the most suitable therapy does not prevent the development of adverse remodeling of the left ventricle for more than 30% of patients suffering anterior wall myocardial infarction (Savoy et al., 2006). In acute myocardial infarction, the transcriptional activity of leukocytes enables the use of easily accessible tissue that is the peripheral blood of the patient to examine the gene expression profile and the search for biomarkers that can be used for the diagnosis, prognosis or monitoring of the course of disease (Sinnaeve et al., 2009).

TIMP-1 belongs to the family of TIMP inhibitors that controls the activity of the matrix metalloproteinases (MMPs) - metallo-dependent peptidases associated with the degradation of the extracellular matrix (ECM). The rearrangement process of the extracellular matrix is of crucial importance in the development of heart failure, and the imbalance of MMPs and TIMP inhibitors may lead to pathological remodelling of the heart connective tissue. Transcription of the gene encoding TIMP1 is induced in response to many cytokines and hormones. It has been shown that *TIMP1* expression at the mRNA and protein levels is increased in the myocardium tissues of patients diagnosed with heart failure (Barton et al., 2003; Polyakova et al., 2011), and increased TIMP1 protein concentration in the blood plasma is a predictive factor of heart failure (Ahmed et al., 2006). In the peripheral blood, leukocytes of patients with acute heart failure, older than 75 years of age, increased levels of *TIMP1* transcription was determined, but similar increase was also observed in a group of elderly patients with infectious diseases or hip fractures (Vo et al., 2011).

RNASE1 encodes a ribonuclease 1, which belongs to the RNase A family mediating the maturation process of nucleic acid precursors and in the cleavage of RNA. RNASE1 is an endonuclease that catalyses the specific hydrolysis of internal phosphodiester bonds in ribonucleic acids from the 3'-end of pyrimidine bases. The enzyme is secreted in the pancreas, where it plays an essential role in the digestion process, but its presence has also been confirmed in other tissues and body fluids that indicate additional physiological functions of RNase 1.

JDP2 is a component of the protein complex forming the transcription factor AP-1 (activating protein-1). Proteins forming AP-1 belong to the following families, Jun (c-Jun, JunB, JunD), Fos (c-Fos, FosB, Fra-1, Fra2), Maf (c-Maf, MafB, MAFA, MAFG/F/K, Nrl) and ATF (ATF2, LRF1/ATF3, B-ATF, JDP1, JDP2). They recognize sequences TRE or CRE (TGA(C/G)TCA) in the promoter regions of many genes involved in the processes of apoptosis, proliferation and differentiation of cells (Shaulian and Karin, 2002). JDP2 acts as a transactivation repressor, where the transactivation is mediated by the Jun family proteins (Katz et al., 2001).

FMN1 is a protein belonging to the formin class that binds actin and is responsible for actin polymerization and microfilament elongation. Formin activity is closely related to processes that require the correct functioning of the actin cytoskeleton, such processes as cytokinesis, endocytosis, maintaining the shape of the cell, its integrity and polarity. The actin skeleton of the heart muscle is also involved in the transmission of mechanical signals (changes of stress in the heart) between cardiomyocytes and extracellular matrix, which results in a change of activity of the RhoA/ROCK signalling pathway and activation of gene expression associated with hypertrophy (Surma et al., 2011). More and more reports show that formins play an important role both as modulators and effectors in the signaling pathway of Rho GTPases (Young and Copeland, 2008).

Various sets of biomarkers for determining the risk of developing heart failure after myocardial infarction are known. US2011/0294683 relates to biomarkers and methods of identifying myocardially-infarcted patients having an increased risk of developing a heart condition.

However, there is still need for developing a method to reliably estimate this risk. This problem is solved by the present invention, which provides suitable methods and kits of biomarkers which were not known in the prior art.

## Summary of the Invention

The present invention is defined in the appended claims.

The present disclosure relates to a composition of transcriptomic biomarkers for diagnosis and/or prognosis of heart failure for post-infarction patients corresponding to the increased expression comparing to non heart failure patient of all the gene transcripts from the group consisting of: *FMN1, RNASE1, JDP2,* and optionally *TIMP1,* more preferably the group consists of: *FMN1, RNASE1, JDP2.*

The present disclosure also relates to a kit for diagnosis and/or prognosis of heart failure for post-infarction patients wherein said kit comprises means for detecting the presence or expression level in a biological sample collected from a patient wherein said detecting comprises detecting all the transcripts of genes from the group consisting of: *FMN1, RNASE1, JDP2,* and optionally *TIMP1.*

Preferably, the means in the kit enable the determination of mRNA expression, preferably isolated from peripheral blood mononuclear cells and/or blood.

Furthermore, the present invention relates to a kit comprising at least two of four genomic probes that are complementary and hybridize the products of expression of *FMN1, RNASE1, TIMP1, JDP2* genes, in preferred embodiment, the products of gene expression *FMN1, RNASE1, JDP2.*

The present disclosure also relates to a method of diagnosing *in vitro* the risk of the patient with post-infarction heart failure, wherein said method comprises: collecting a biological sample from the patient after myocardial infarction, determining the gene expression profile in leukocytes, and determining the risk of heart failure based on the obtained profile.

In a preferred embodiment the method comprises measuring, in a sample collected from a subject, the expression of at least two genes transcripts from the group consisting of: *FMN1, RNASE1, TIMP1, JDP2,* or the expression of at least one gene transcript from the group consisting of: *FMN1, RNASE1, JDP2*

In a further preferred embodiment the biological sample is blood, blood cells, serum, plasma or preferably peripheral blood mononuclear cells, and preferably the expression level of mRNA biomarkers is determined. In a preferred embodiment, the biomarker is determined by PCR, preferably quantitative RT-PCR. According to the invention an increased level of expression of biomarkers correlates with disease condition.

In a still further preferred embodiment, the detection of biomarker expression is carried out by immunoassay in which the risk is diagnosed based on the sample binding or absence of binding to the panel.

The present disclosure also relates to a method for determining the markers in samples of biological material collected from a patient, wherein the said method comprises the determination of levels of expression of at least one gene transcript from the following group: *FMN1, RNASE1, TIMP1, JDP2,* or at least two genes from the following group of transcripts: *FMN1, RNASE1, TIMP1, JDP2.*

The present disclosure also relates to the use of biomarkers for the diagnosis of developing post-infarction heart failure wherein the expression levels of at least two gene transcripts from the group consisting of: *FMN1, RNASE1, TIMP1, JDP2,* or at least one gene from the group of transcripts consisting of: *FMN1, RNASE1, JDP2* are determined in samples of biological material collected from a patient.

The present disclosure also relates to the prevention of heart disease based on the expression profile of genes in leukocytes in acute myocardial infarction, preferably prophylaxis is based on the diagnosis of risk by the invention.

The present disclosure also relates to a method for determining predisposition to (predicting the risk of) heart failure for post-infarction patients, wherein said method comprises the determination of markers in samples of biological material collected from a patient. In particular, said method comprises the determination of the levels of expression of two genes from the group of transcripts consisting of: *FMN1, RNASE1, TIMP1, JDP2,* or at least one gene from the transcript group consisting of: *FMN1, RNASE1, JDP2.*

Furthermore, the present disclosure also relates to a method of optimizing the treatment of cardiac patients based on the result of determining the markers in biological material samples collected from a patient, wherein said method comprises determining the levels of expression of two genes from the transcript group consisting of: *FMN1, RNASE1, TIMP1, JDP2,* or at least one gene transcripts from the group consisting of: *FMN1, RNASE1, JDP2.* Moreover, the present disclosure also relates to the method of individual risk assessment and early identification of patients for the likelihood of the development of heart failure for a post-infarction patient, wherein said method is based on the expression profile of biomarkers according to the means of the invention.

## Brief description of the drawings

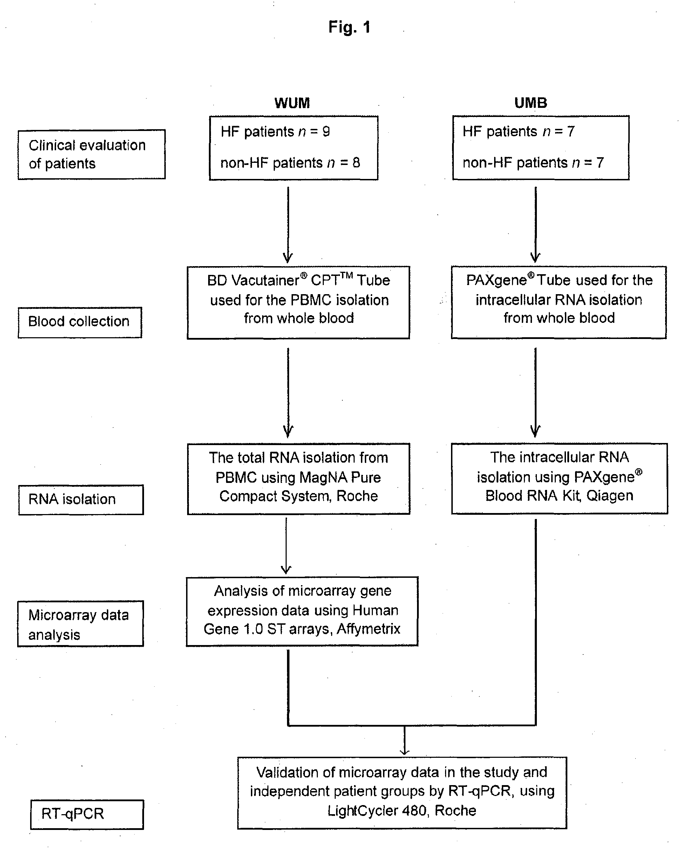

Fig 1 is a scheme for the methodology based on the studies described in the examples;

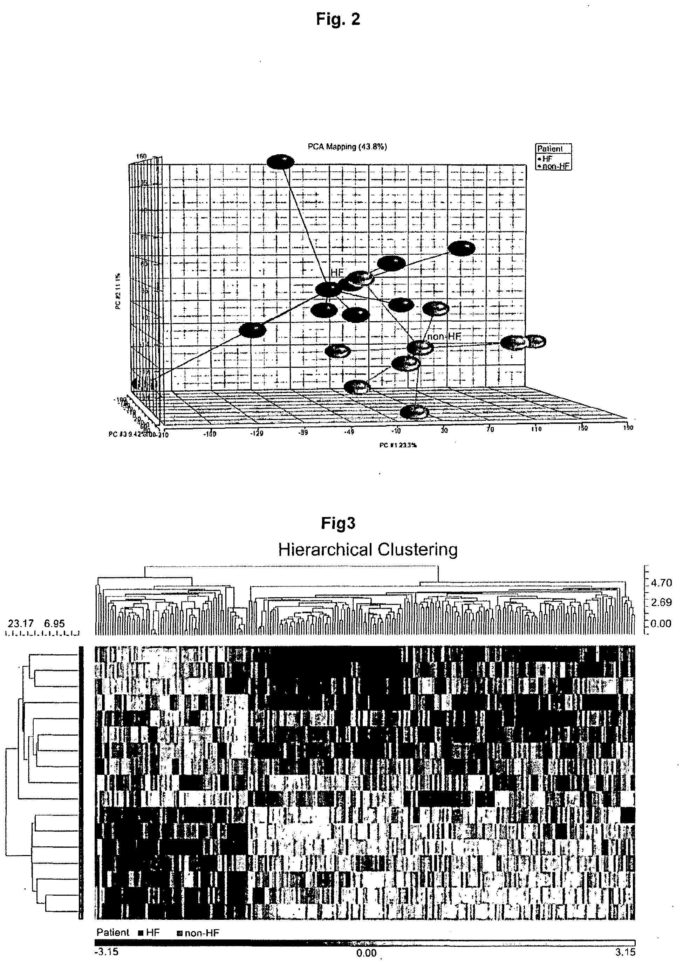

Fig. 2 shows the PCA analysis of the tested samples.
Fig, 3. is the heat map showing the levels of transcripts that differentiate between the studied groups.

## Detailed description of the invention

Biomarkers, selected on the basis of high-throughput transcriptomic experiments, which relate to changes in mRNA expression correlate with the prognostic value of post-infarction patients

The present disclosure relates to a method for predicting the risk of heart failure development for post-infarction patients, comprising determination of the increased level of expression of at least two genes from the following transcript group: *FMN1, RNASE1, TIMP1, JDP2* in a sample from a post-infarction patient. Briefly, the method comprises isolating RNA from blood collected from a patient, followed by cDNA synthesis, determining the expression level of genes by RT-qPCR (quantitative reverse transcription polymerase chain reaction) and analysing the relative changes in expression levels of particular genes by comparing mRNA levels of such genes for post-infarction patients relative to the mRNA levels of the same genes for healthy subjects and then normalizing the data with respect to mRNA levels of the selected reference genes taking into account the differences in the amplification efficiency of particular genes.

The present disclosure also relates to a diagnosis method and/or determination of the prognosis for the specific patient on the basis of the expression of mRNA of selected transcripts determined within the RNA isolated from peripheral blood mononuclear cells (PBMC) and whole blood. The increase or decrease in the expression level of selected genes correlates with the disease condition when compared to the level of gene expression for the subject with no pathological condition. The present disclosure also relates to complementary risk assessment and early identification of post-infarction patients with high risk of heart failure. The expression profiles of selected genes are generated using RT-qPCR and may be used in conjunction with other prognostic and diagnostic methods.

The invention has been described with reference to the examples provided below, which are provided merely to allow the skilled person to carry out the invention, but the scope of protection is set forth in the claims.

## Examples

*Bioassays*

Methods disclosed herein are based on the study of the expression of biomarkers in a sample collected from the patient. The term "sample" is meant to relate to biological material obtained from the patient. The preferred sample is a blood sample collected from a patient, wherein the said sample is then processed in accordance with the methodology of the invention. The samples can be processed by means of incubation, centrifugation, precipitation, concentration, dilution. The type of procedures used may depend on the method for collection of blood, and the length of time available for RNA isolation. The procedure types used here do not affect the further processing of the sample.

*Determination of gene expression*

According to this disclosure, mRNA expression of at least two genes selected from the group consisting of the following genes *FMN1, RNASE1, TIMP1, JDP2* are determined:

**Summary of the data on the studied transcripts and reference gene HPRT1**

| Gene ID | TIMP1 |
| --- | --- |
| Synonyms | CLGI; EPA; EPO; HCl; TIMP |
| Full name | Metallopeptidase inhibitor 1 |
| The identification number RefSeq (NCBI Reference Sequence) | NM_003254 |
| HGNC identification number | 11820 |
| NCBI identification number GeneID | 7076 |
| Subject | Homo sapiens |

| Gene ID | RNASE1 |
| --- | --- |
| Synonyms | RIB1; RNS1 |
| Full Name: | ribonuclease 1 (pancreatic), RNase A family, 1 (pancreatic) |
| The identification number RefSeq (NCBI Reference Sequence) | NM_198232 |
| HGNC identification number | 10044 |
| NCBI identification number GeneID | 6035 |
| Subject | Homo sapiens |

| Gene ID | JDP2 |
| --- | --- |
| Synonyms | JUNDM2 |
| Full name | Jun dimerization protein 2 |
| The identification number RefSeq (NCBI Reference Sequence) | NM_001135049 |
| HGNC identification number | 17546 |
| NCBI identification number GeneID | 122953 |
| Subject | Homo sapiens |

| Gene ID | FMN1 |
| --- | --- |
| Synonyms | FMN, LD |
| Full name | Formin 1 |
| The identification number of RefSeq (NCBI Reference Sequence) | NM_001103184 |
| HGNC identification number | 3768 |
| NCBI identification number GeneID | 342184 |
| Subject | Homo sapiens |

| Gene ID | HPRT1 |
| --- | --- |
| Synonyms | HGPRT, HPRT |
| Full name | hypoxanthine phosphoribosyltransferase 1 |

(continued)

| Gene ID | HPRT1 |
| The identification number RefSeq (NCBI Reference Sequence) | NM_000194 |
| HGNC identification number | 5157 |
| NCBI identification number GeneID | 3251 |
| Subject | Homo sapiens |

Numerous methods for determining the level of biomarkers in the analyzed samples can be used. Gene expression can be tested, for example, using microarrays or RT-qPCR.

Typically, the microarray is a glass or plastic plate on which the DNA sequences (probes) are printed, allowing the study of the gene expression of tens of thousands of genes. The detection method based on matrix technology generally consists of the following steps: (1) isolating RNA from the samples to be tested and cDNA synthesis by reverse transcription (2) hybridization of the labelled RNA with probes and then washing the matrix to remove unbound sequence (3) scanning an image of the matrix (4) qualitative and statistical analysis of the results. Many types of methods based on matrix techniques are now available.

Alternatively, the expression levels of mRNA biomarkers according to the invention can be measured by RT-qPCR. According to this method, the patient's blood RNA is collected and isolated, followed by cDNA synthesis, then determination of the expression level of the gene by RT-qPCR and analysis of the relative changes in expression of particular genes by comparing the mRNA levels of genes for the patients with disease condition with data relative to the mRNA levels of the same genes in healthy subjects and normalizing said data with respect to mRNA levels of the selected reference genes taking into account the differences in the efficiency of amplification of particular genes.

*Patients*

Coronary artery disease, which is one of the most common form of cardiovascular pathology, is the main cause of cardiac failure (HF). Myocardial infarction is an event in coronary artery disease, particularly often leading to the development of HF. The development of heart failure after acute myocardial infarction is directly related to adverse remodelling of the left ventricle.

The study group comprised of 17 patients with a first episode in a lifetime of acute myocardial infarction with ST-segment elevation, characterized clinically, from the Medical University of Warsaw (WUM). A separate group of patients with similar clinical characteristics consisted of 14 patients from the Medical University of Bialystok (UMB). On the basis of clinical parameters, both groups were divided into a group of patients who developed post-infarction heart failure (HF) in the 6-month follow-up, and a group of patients that did not develop post-infarction heart failure (non-HF) (in the 6-month follow-up). The clinical parameters of the patients are shown in Table 1.

Table 1. Characteristics of the study and validation groups

| Parameter | Study group | | Validation group | |
|---|---|---|---|---|
| | HF($n = 9$) | non-HF ($n = 8$) | HF($n = 7$) | non-H F ($n = 7$) |
| Age (years)* | 60.2±14.1 | 51.7 ±7.2 | 59.7±9.4 | 56.7±13.2 |
| Females/Males (*n/n*) | 3/6 | 1/7 | 0F/7 | 0/7 |
| BMI* | 26.8±3.1 | 25.5±1.6 | 30±3.7 | 26.1±4.2 |
| Hypertension, *n* (%) | 3 (33.3 %) | 1 (12.5%) | 5 (71.4 %) | 5 (71.4 %) |
| Diabetes, *n* (%) | 2 (22.2 %) | 1 (12.5 %) | 2 (28.6 %) | 0 (0 %) |
| Hyperlipidemia, *n* (%) | 5 (55.5 %) | 4 (50 %) | 5 (71.4 %) | 5 (71.4 %) |
| HF(*n* = 9) | non-HF (*n* = 8) | HF(*n* = 7) | non-HF (*n* = 7) | |
| Previous myocardial infarction, *n* (%) | 0 (0 %) | 0 (0 %) | 0 (0 %) | 0 (0 %) |
| Smoking cigarettes, *n* (%) | 3 (33.3 %) | 5 (62.5 %) | 3 (42.9 %) | 5 (71.4 %) |
| LVEF (%)* | 39.3±8.4** | 66.7±1.9** | 28.86±6.3** | 57.7±4.7** |
| NT-proBNP (pg/ml)* | 918.3±848.5** | 62.0±14.1** | 1960.9±3421.9** | 119.4±118.9** |

(continued)

| Treatment | | | | |
|---|---|---|---|---|
| ACEI/ARB | 9 (100%) | 8 (100 %) | 7 (100 %) | 7 (100 %) |
| Beta-blockers | 9 (100 %) | 8 (100%) | 7 (100 %) | 7 (100 %) |
| Statins | 9 (100 %) | 8 (100 %) | 7 (100 %) | 7 (100 %) |

BMI - Body Mass Index
LVEF - Left Ventricular Ejection Fraction
NT-proBNP - N-terminal pro-B-type Natriuretic Peptide ACEI/ARB - angiotensin-converting enzyme inhibitors /angiotensin receptor blockers
*Results are expressed as mean $\pm$ standard deviation or as the % of number of patients.
** The LVEF, NT-proBNP values 6 months after myocardial infarction.

The group of patients from WUM was analysed using high-throughput methods; on the basis of these analyses a panel with 4 transcripts was selected. The transcripts were validated by RT-qPCR and additionally tested on an independent group of patients from UMB. Additionally, in both groups of patients, different methods of blood collection and RNA isolation were used. The method used for patients from WUM requires very rapid isolation of PBMC (up to 1 hour), while the method used for patients from UMB allows transportation of a blood sample and RNA isolation at a convenient time. We have found that the time and method for obtaining material for further testing does not affect the RNA quality and yield of the reaction.

Biomarkers according to the present disclosure enable supplement of risk assessment and early identification of patients with high risk of post-infarction heart failure. The studied biomarkers are of high prognostic value because assessment thereof makes it possible to predict the risk of developing heart failure.

The following are detailed examples of the application.

**Example 1**

Methods

Characteristics of the patients

The studies were designated to determine the level of gene expression in circulating blood of patients after myocardial infarction with elevated ST-segments that were treated with primary percutaneous coronary intervention. The study was approved by the local Ethics Committee of the Medical University of Warsaw and all participants gave written informed consent. Peripheral blood samples were collected from 17 patients from WUM on the 1st day of AMI (admission), after 4-6 days (discharge), 30-35 days, and 6 months after myocardial infarction, patients were clinically monitored. The characteristics of patients was based on clinical assessment parameters. All patients were treated on the 1st day of AMI with primary coronary angioplasty, as well as by administration of aspirin, clopidogrel, angiotensin-converting enzyme inhibitors /angiotensin receptor blockers (ACEI/ARB), beta-blockers and statins. Patients were selected into a group of patients who developed the post-infarction heart failure (HF) and a group of patient who did not develop the post-infarction heart failure (non-HF) on the basis of ejection fraction of left ventricle (LVEF) and biochemical marker NT-proBNP (N-terminal pro-B-type Natriuretic Peptide) 6 months after the myocardial infarction. For patients in the HF group, the mean value of LVEF was 39.3% and the average value of NT-proBNP was 918 (pg/ml), while for non-HF patients, the value of LVEF was 66.7% and NT-proBNP level was 62 (pg/ml).

*RNA isolation*

Whole blood samples from patients from WUM were collected into Vacutainer®CPT™ BD tubes (Becton, Dickinson and Company, NY, USA). Isolation of PBMC was performed by centrifuging and washing the cells in physiological buffered saline (PBS). Then, PBMC were suspended in RNAlater® solution (Ambion Inc., TX, USA) and stored at -80°C. RNA was isolated with MagNA Pure® Compact apparatus using the reagent kit MagNA Pure Compact RNA Isolation Kit. (Roche Diagnostics GmbH, Mannheim, Germany). The quality of the isolated RNA was assessed using a Bioanalizer (Agilent 2100 600 Bioanalizer and RNA Nano Assay kit, Agilent, Agilent technologies, Pao Alto, CA, USA). All samples had a high integrity of RNA ratio (called RNA Integrity Number) - above 8, allowing the synthesis reaction of cRNA and cDNA in microarray analysis.

*Microarray analysis*

The experiment was performed on an Affymetrix microarray GeneChip Human Gene 1.0 ST (Affymetrix, Inc., Santa Clara, CA, USA). Synthesis reactions of cRNA, cDNA, fragmentation and labeling were performed using an Ambion WT Expression Kit (Ambion Inc., TX, USA) and the Affymetrix GeneChip WT Terminal Labeling Kit (Affymetrix, Inc., Santa Clara, CA, USA). To monitor the progress of the reaction: Affymetrix GeneChip Poly-A RNA Control Kit and the Affymetrix GeneChip Hybridization Controls (Affymetrix, Inc., Santa Clara, CA, USA) were used. Hybridization was conducted for 17 h at 45°C (Affymetrix GeneChip Hybridization Oven 640), followed by rinsing on the Affymetrix GeneChip Fluidics Station 450, and scanning using the Affymetrix GeneArray Scanner 3000 GCS (apparatus and reagents: Affymetrix, Inc., Santa Clara, CA, USA). Analysis of the technical parameters of the plates was performed using Affymetrix Expression Console software 1.2.1.20.

*Statistical analysis of microarray results*

The program Partek Genomics® (Partek Inc., St. Louis, MO, USA) was used to analyse the transcriptomic results. "Raw" fluorescence data from each microarray were normalized using RMA (Robust Multi-array Analysis). To calculate the changes in the expression of genes of interests between samples (Fold Change) and the significance of differences in the gene expression, the analysis of variance (ANOVA) was used. The functional analysis was performed using Ingenuity software (Ingenuity Systems, Inc. Redwood City, CA, USA).

*Quantitative RT-qPCR*

Quantitative RT-qPCR was performed in 3 steps:

1) Synthesis of cDNA by reverse transcription

The isolated RNA was used for cDNA synthesis by reverse transcription using QuantiTect® Reverse Transcription Kit (QIAGEN, Germany). For the synthesis of cDNA, 200 ng of RNA (PBMC) was used, 2 $\mu$l of 7x gDNA Wipeout buffer and up to 14 $\mu$l of RNase-Free Water. Then, the prepared samples were incubated in a water bath at 42°C for 30 min.(DNase step). Then, 4 ul of 5x Quantiscript RT buffer, 1 $\mu$l of a mixture of primers RT Primer Mix and 1 $\mu$l Quantiscript Reverse Transcriptase were added to the reaction mixture. The reverse transcription reaction was performed in a personal Mastercycler (Eppendorf, Hamburg, Germany) at 42°C for 30 min.; followed by denaturation of the reaction at 95°C for 3 min.

2) Gene expression analysis by real time RT-qPCR

Real time RT-qPCR was used to analyse the expression of the genes of interest. For conducting the RT-qPCR reaction, LightCycler 480 SYBR Green I Master assembly was used with a 96-well plates LightCycler® (Roche, Germany) and the apparatus LightCycler® 480 System (Roche, Germany). The mixture composition in one sample (10 $\mu$l) was as follows: 2 $\mu$l of $H_2O$, and 1 $\mu$l of each diluted primer 20x (5 pmol/$\mu$l), 5 $\mu$l of 2x LightCycler® 480 SYBR Green I Master and 1 $\mu$l of cDNA out of 100 $\mu$l. In each experiment, a negative control was made, consisting of a Master Mix, primers, water with no added cDNA template. For quantitative measurements, the following four-step protocol was used:

- 10-minute pre-incubation at 95°C

- 45 cycles of amplification, each cycle consisted of four stages:

    1. denaturation at 95°C/10s

    2. annealing (annealing temperature for *FMN1* 56°C/5 seconds, *TIMP1* 54°C/5 seconds, *RNASE1* 58°C/5 seconds, *JDP2* 58°C/5 seconds, *HPRT1* 55°C/5s)

    3. extension at 72°C (extension time for *FMN1* 9s, for *TIMP1* 6s, for *RNASE1* 9s, for *JDP2* 7s, and for *HPRT1* 5s)

- Melting curve 95°C/5 sec, 65°C/1 min and then slowly heating the samples at 0.1°C/s to 97°C with continuous fluorescence measurement

- cooling at 40°C/10s

The number of copies of the nucleic acid molecules of the analysed genes was monitored in each cycle of the amplification reaction. The number of PCR cycles at which the fluorescence rises appreciably, defined as the threshold ($C_P$ - crossing point), was used to calculate the amount of test molecules present in the mixture at the start of the reaction. For each sample, the $C_P$ value for gene housekeeping *HPRT1* - an endogenous control - was used to normalize the level of gene expression. For each gene amplification, the efficiency was estimated for transcripts analysed on the basis of the amplification curves.

The specificity of the amplified product was verified by melting curve analysis and 3 % agarose gel electrophoresis. Each measurement of gene expression for each patient was carried out in triplicate.

Table 2. Primer sequences and reaction conditions for RT-qPCR

| Gene ID | Product length | The annealing temperature | Elongation time at 72°C | Amplification efficiencies for specific | Primer sequence |
|---|---|---|---|---|---|
| *FMN1* | 208 | 56°C | 9s | 2.00 | F: 5' CCAAGAGGATGAGCTGGTTA 3'<br>R: 5' AGCTCGCGTGATGATCTCTA 3' |
| *RNASE1* | 172 | 58°C | 9s | 2.00 | F: 5' GTCCGGCTCCTTCTGCTTGT 3'<br>R: 5'GTGTCATATTCCGGCGCCTC 3' |
| *TIMP1* | 148 | 54°C | 6s | 1.82 | F: 5' GCTTCTGGCATCCTGTTGTT 3'<br>R: 5' AGGTGGTCTGGTTGACTTCT 3' |

| Gene ID | Product length | The annealing temperature | Elongation time at 72°C | Amplification efficiencies for specific | Primer sequence |
|---|---|---|---|---|---|
| *JDP2* | 188 | 58°C | 7s | 2.00 | F: 5' TACGCTGACATCCGCAACCT 3'<br>R: 5' AACTCCGTGCGCTCCTTCTT 3' |
| *HPRT* | 102 | 55°C | 5s | 1.80 | F: 5' TGACCTTGATTTATTTTGCATACC 3<br>R: 5'CGAGCAAGACGTTCAGTCCT 3' |

3.) The mathematical model used to estimate the relative mRNA levels of specific genes

To determine the changes in the amount of mRNA of the studied genes, a Pfaffl's model was used wherein the mRNA level of the genes of interest are normalized in relation to the mRNA levels of the selected reference genes, taking into account the differences in the amplification efficiency of individual transcripts. The author of the model presents a calculation of the relative expression level of the studied genes using the following formula:

$$\text{Ratio} = (E_{target})^{\Delta CP\ target\ (control-sample)} / (E_{ref})^{\Delta CP\ ref\ (control-sample)}$$

Ratio - the level of the -investigated transcript in an unknown sample expressed as a multiple of the calibrator

$E_{target}$ - efficiency of a target gene transcript

$E_{ref}$ - efficiency of a reference gene transcript

$\Delta CP$ target (control-sample) - the difference in the mean values of the target gene transcript Cp

$\Delta CP$ ref (control-sample) - the difference in the mean values of the reference gene transcript Cp

Levels of mRNA of the genes of interest were normalized to mRNA levels of the *HPRT1* gene. To estimate the relative mRNA levels of each gene of interest, Relative Expression Software Tool - version 2 was used. This program allows the comparison of the mRNA levels from two groups (control and test) and testing the statistical significance of the reported differences in mRNA levels using Pair Wise Fixed Reallocation Test.

**Example 2**

Methods

Characteristics of the patients

The studies were conducted in order to verify the detected biomarkers for post-infarction heart failure with an independent group of patients from the Medical University of Bialystok (UMB). 14 patients after myocardial infarction event with acute ST-segment elevation and treated with primary percutaneous coronary intervention, agreed to participate in a research study by signing the informed consent certificates. The study was approved by the local Ethics Committee of the Medical University of Bialystok and all participants gave written informed consent. The characteristics of the patients was based on an assessment of their clinical parameters. The blood samples were collected from patients on the 1st day of AMI (admission), then after 4-6 days (discharge), and 30-35 days, and 6 months after myocardial infarction, patients were clinically monitored similarly to patients from WUM. Analogously, all patients were treated in the first day of primary coronary angioplasty, as well as administration of aspirin, clopidogrel, angiotensin-converting enzyme inhibitors/angiotensin receptor blockers (ACEI/ARB), beta-blockers and statins. The selection of the patients into a specific group of patients with the developing post-infarction heart failure (HF) and a group without the developing post-infarction heart failure (non-HF) was conducted on the basis of ejection fraction of left ventricle (LVEF) and biochemical marker NT-proBNP (N-terminal pro-B-type Natriuretic Peptide) 6 months after the myocardial infarction. For patients assessed to the HF group the mean value of LVEF was 28.9%, the mean value of NT-proBNP was 1960.9 pg/ml and for patients non-HF, LVEF was 57.7%, and NT-proBNP - 119.4 pg/ml.

*RNA isolation*

Whole blood samples from patients from UMB were collected into tubes PAXgene™ (Qiagen, Germany), followed by incubation for 2 hours at room temperature. Subsequently tubes with blood were transported (at + 4°C) to the laboratory for further study. RNA was isolated 24 hours after the collection of the samples. RNA isolation kit PAXgene Blood RNA was used for RNA isolation. The system was used in accordance with the procedure specified by the manufacturer. The quality of the isolated RNA was assessed using the Bioanalizer (Agilent 2100 600 Bioanalizer and RNA Nano Assay kit, Agilent, Agilent Technologies, Pao Alto, CA, USA).

*Quantitative RT-PCR*

Quantitative RT-PCR was performed according to the methodology developed for RNA obtained from patients from WUM (Example 1).

**RESULTS**

*Patients*

The informed consent agreement and blood samples were obtained from 31 patients from two independent clinical centers: Medical University of Warsaw (n = 17) and the Medical University of Bialystok, n = 14). According to some clinical parameters (LVEF - ejection fraction of left ventricle and NT-proBNP - N-terminal pro-B-type Natriuretic Peptide) examined 6 months after the onset of myocardial infarction in patients, who were divided into two groups: patients who developed post-infarction heart failure (HF), and those non-HF - in case of these patients, the myocardial infarction did not lead to the development of heart failure.

*Selection of genes associated with the development of post-infarction heart failure*

In order to identify genes whose expression level changes may be a parameter for the development of heart failure after an acute myocardial infarction. Microarray analysis was conducted with the samples collected from patients from WUM. The raw microarray data was normalized using the RMA method (Robust Multi-Array Analysis). An unsupervised analysis of microarray data using Principal Component Analysis (PCA) was conducted. The PCA plot demonstrated a distinct separation between HF and non-HF patients.

Analysis of variance (ANOVA) was used for the analysis of differentially expressed genes in the test samples, using the following cut-off criteria $p < 0.05$ and fold change $\pm > 1.5$. The list of 225 genes (107 annotated and 118 without annotations) was received.

The following table presents a list of differentially expressed genes with annotation: s

| Gene Symbol | RefSeq | p-value (Patient) | Fold-Change (HF vs non-HF) |
|---|---|---|---|
| ABCD2 | NM_005164 | 0.0469917 | -1.70971 |
| ADAMTS2 | NM_014244 | 0.0448791 | 1.96014 |
| ADM | NM_001124 | 0.0307457 | 1.52064 |
| AK5 | NM_174858 | 0.0311163 | -1.93798 |
| AMY2B | NM_020978 | 0.01142 | -1.59821 |
| ANKRD36B | NM_025190 | 0.038301 | -1.54762 |
| APBB2 | NM_004307 | 0.0231786 | 1.59166 |
| AREG | NM_001657 | 0.0268264 | 1.8885 |
| AREG | NM_001657 | 0.0357281 | 2.03204 |
| BANK1 | NM_017935 | 0.0495889 | -1.58347 |
| C6orf25 | NM_138277 | 0.0409132 | 1.63637 |
| C6orf25 | NM_138277 | 0.0430783 | 1.65733 |
| C6orf25 | NM_138277 | 0.039982 | 1.66355 |
| C9orf95 | NR_023352 | 0.0295803 | -1.63004 |
| CD24 | NM_013230 | 0.0404677 | -1.74763 |
| CD28 | NM_006139 | 0.034903 | -1.63474 |
| CDA | NM_001785 | 0.0133897 | 1.60735 |
| CLU | NM_001831 | 0.0380488 | 1.86459 |
| COBLL1 | NM_014900 | 0.024395 | -1.5327 |
| CR2 | NM_001006658 | 0.0168323 | -1.55197 |
| CRISPLD2 | NM_031476 | 0.0278502 | 1.52319 |
| CSGALNACT1 | NM_018371 | 0.0213203 | -1.50285 |
| DOCK9 | NM_015296 | 0.0316927 | -1.56242 |
| DPP4 | NM_001935 | 0.0413474 | -1.58096 |
| DSC1 | NM_024421 | 0.0178057 | -1.70612 |
| EPHX2 | NM_001979 | 0.0196545 | -1.59913 |
| FAM113B | BC008360 | 0.009905 | -1.52221 |
| FAM153B | NM_001079529 | 0.0198199 | -1.62746 |
| FAM153B | NM_001079529 | 0.0296789 | -1.5372 |

(continued)

| Gene Symbol | RefSeq | p-value (Patient) | Fold-Change (HF vs non-HF) |
|---|---|---|---|
| FAM153C | NM_001079527 | 0.0323118 | -1.55234 |
| FCRL1 | NM_052938 | 0.0340379 | -1.65221 |
| FCRL2 | NM_030764 | 0.0132842 | -1.73915 |
| FLT3 | NM_004119 | 0.0329164 | 2.08236 |
| FMN1 | ENST00000414268 | 0.00958055 | 2.79016 |
| FOS | NM_005252 | 0.0352322 | 1.6684 |
| FOSB | NM_006732 | 0.0159216 | 1.99281 |
| FSTL1 | NM_007085 | 0.0323571 | 1.58923 |
| G0S2 | NM_015714 | 0.0204903 | 1.55081 |
| GCET2 | NM_001008756 | 0.0179733 | -1.51726 |
| GPER | NM_001039966 | 0.0293458 | 1.51442 |
| GPR15 | NM_005290 | 0.0298187 | -3.08618 |
| GSDMB | NM_001165958 | 0.00762663 | -1.72045 |
| HIVEP2 | NM_006734 | 0.00683168 | -1.54514 |
| HMOX1 | NM_002133 | 0.0130014 | 1.51249 |
| HRH4 | NM_021624 | 0.0415242 | -1.57451 |
| ICOS | NM_012092 | 0.0334686 | -1.61596 |
| IFNGR1 | NM_000416 | 0.0265609 | 1.541 |
| IKZF2 | NM_016260 | 0.0478205 | -1.56544 |
| IL1R2 | NM_004633 | 0.0394166 | 3.10348 |
| IL2RA | NM_000417 | 0.0302936 | -1.53516 |
| INPP4B | NM_003866 | 0.0494143 | -1.5426 |
| ITGA6 | NM_000210 | 0.0261737 | -1.62354 |
| JDP2 | NM_001135049 | 0.0107536 | 1.8113 |
| KCTD12 | NM_138444 | 0.012041 | 1.53324 |
| KIAA0748 | NM_001098815 | 0.0146606 | -1.51695 |
| LHFPL2 | NM_005779 | 0.0256441 | 1.56697 |
| LOC100131581 | AK092544 | 0.0129399 | -1.69531 |
| LRRN3 | NM_001099660 | 0.0235208 | -2.5529 |
| LYVE1 | NM_006691 | 0.0378372 | 1.73741 |
| MAL | NM_002371 | 0.0273941 | -1.59613 |
| MARS2 | NM_138395 | 8.70681e-007 | -1.54351 |
| MFSD2B | NM_001080473 | 0.0456674 | 1.5212 |
| MGAM | NM_004668 | 0.0273156 | 1.89092 |
| MS4A1 | NM_152866 | 0.0428405 | -1.78338 |
| MS4A3 | NM_006138 | 0.0469504 | -1.61668 |
| NAMPT | NM_005746 | 0.0360701 | 1.53312 |
| NAMPT | NM_005746 | 0.0258243 | 1.583 |
| NELL2 | NM_006159 | 0.0192659 | -1.78015 |
| NFIL3 | NM_005384 | 0.0241924 | 1.69166 |
| NRGN | NM_006176 | 0.0179662 | 1.6994 |
| OR52N4 | NM_001005175 | 0.0244699 | -1.64949 |
| OSBPL10 | NM_017784 | 0.013799 | -1.61061 |
| P2RY10 | NM_014499 | 0.0310185 | -1.53974 |
| PER1 | NM_002616 | 0.018923 | 1.63349 |
| PGA3 | NM_001079807 | 0.0116366 | 1.7659 |
| PGA4 | NM_001079808 | 0.0122908 | 1.84885 |
| PGA5 | NM_014224 | 0.0123735 | 1.82358 |
| PVALB | NM_002854 | 0.0371886 | 1.83557 |
| RASGRF2 | NM_006909 | 0.0341977 | -1.5221 |

(continued)

| Gene Symbol | RefSeq | p-value (Patient) | Fold-Change (HF vs non-HF) |
|---|---|---|---|
| RASGRP3 | NM_170672 | 0.0309359 | -1.55868 |
| RIC3 | NM_024557 | 0.0260037 | -1.55672 |
| RNASE1 | NM_198232 | 0.0227935 | 1.95369 |
| SLED1 | NR_003542 | 0.028744 | 2.22292 |
| SNORA22 | NR_002961 | 0.0192458 | -1.78864 |
| SNORA61 | NR_002987 | 0.0371973 | -1.5556 |
| SNORD116-24 | NR_003338 | 0.0230589 | -1.86354 |
| SNORD28 | NR_002562 | 0.0296354 | -1.68784 |
| SNORD42A | NR_000014 | 0.0172731 | -1.59266 |
| SNORD42B | NR_000013 | 0.0120945 | -1.5425 |
| SNORD47 | NR_002746 | 0.0277813 | -1.75842 |
| SPARC | NM_003118 | 0.0459948 | 1.77743 |
| SPIC | NM_152323 | 0.00905216 | 1.63721 |
| ST14 | NM_021978 | 0.0239803 | 1.53647 |
| STAP1 | NM_012108 | 0.0219813 | -1.77671 |
| TAS2R4 | NM_016944 | 0.00342938 | -1.57668 |
| TCN2 | NM_000355 | 0.0283251 | 1.87292 |
| TIMP1 | NM_003254 | 0.00838666 | 1.50224 |
| TMEM194B | NM_001142645 | 0.0194176 | -1.594 |
| TPST1 | NM_003596 | 0.028574 | 2.0356 |
| TRAV8-3 | X58769 | 0.0357429 | -1.59254 |
| TSHZ2 | ENST00000371497 | 0.0128499 | -1.77547 |
| TSHZ2 | NM_173485 | 0.016395 | -1.59924 |
| VSIG1 | NM_001170553 | 0.0389136 | -1.53166 |
| ZNF253 | NM_021047 | 0.0240569 | -1.52272 |
| ZNF429 | NM_001001415 | 0.000133358 | -1.97188 |
| ZNF493 | NM_001076678 | 0.0110629 | -1.50734 |
| ZNF860 | NM_001137674 | 0.0329027 | -1.67335 |

The hierarchical cluster analysis (heat map) of gene expression value for 225 transcripts (HF vs non-HF) showed a different transcription profile that differentiates the studied groups of patients.

*Increased expression of selected transcripts (biomarkers) correlated with the development of heart failure*

Quantitative RT-PCR was performed in order to validate the microarray data (from WUM) and to confirm the results for an independent group of patients (from UMB). Using the *HPRT1* gene as a housekeeping gene, it was demonstrated that the expression of the following genes, *FMN1, RNASE1, TIMP1, JDP2* was increased for HF patients compared to non-HF patients.

Table 3. Validation results of the microarray results for a group from WUM

| Gene Symbol | Gene Name | Sequence reference number (RefSeq) | Results from microarray | RT-qPCR* |
|---|---|---|---|---|
| *FMN1* | Formin 1 | NM_001103184 | 2.79 $p = 0.01$ | 2.15 $p = 0.023$ |
| *RNASE1* | Ribonuclease, RNase A family, 1 (pancreatic) | NM_198232 | 1.95 $p = 0.02$ | 3.76 $p = 0.006$ |
| *TIMP1* | TIMP metallopeptidase inhibitor 1 | NM_003254 | 1.50 $p = 0.01$ | 1.56 $p = 0.003$ |

(continued)

| Gene Symbol | Gene Name | Sequence reference number (RefSeq) | Results from microarray | RT-qPCR* |
|---|---|---|---|---|
| *JDP2* | Jun dimerization protein 2 | NM_001135049 | 1.81 $p$ = 0.01 | 1.85 $p$ = 0.008 |
| *results normalized to HPRT1 gene* | | | | |

Tabela 4. Validation results for the independent group of patients (UMB)

| Gene Symbol | Gene Name | Sequence reference number (RefSeq) | RT-qPCR* |
|---|---|---|---|
| *FMN1* | Formin 1 | NM_001103184 | 1.91 p=0.042 |
| *RNASE1* | Ribonuclease, RNase A family, 1 (pancreatic) | NM_198232 | 3.72 p=0.022 |
| *TIMP1* | TIMP metallopeptidase inhibitor 1 | NM_003254 | 1.77 p=0.003 |
| *JDP2* | Jun dimerization protein 2 | NM_001135049 | 1.517 p=0.037 |
| *results normalized to HPRT1 gene* | | | |

Modeling of a gene network was performed using Ingenuity Pathway Analysis (IPA). Direct interactions are indicated by solid lines, while the indirect interactions are indicated using dashed lines. On the basis of the IPA literature, the database interaction network was linked to the incidence of cardiac hypertrophy symptoms.

**Final conclusions**

1. The conducted research confirms the existence of a correlation between the expression levels of transcripts: *FMN1, RNASE1, TIMP1, JDP2* and prognostic value of post-infarction patients.

2. The obtained results allow diagnosis and/or determination of the prognosis for the patient based on the expression of the selected mRNA transcripts determined in RNA samples.

References:

1. Czech M, Opolski G, Zdrojewski T, Dubiel JS, Wizner B, Bolisęga D, Fedyk-Łukasik M, Grodzicki T. The costs of heart failure in Poland from the public payer's perspective. Polish programme assessing diagnostic procedures, treatment and costs in patients with heart failure in randomly selected outpatient clinics and hospitals at different levels of care: POLKARD. Kardiol Pol. 2013;71(3):224-32.

2. Anwaruddin S, Askari AT, Topol EJ. Redefining risk in acute coronary syndromes using molecular medicine. J Am Coll Cardiol. 2007; 49(3):279-89.

3. Horne BD, Carlquist JF, Muhlestein JB, Nicholas ZP, Anderson JL; Intermountain Heart Collaborative Study Group. Associations with myocardial infarction of six polymorphisms selected from a three-stage genome-wide association study. J. Am Heart 2007; 154(5):969-75.

4. Madjid M, Awan I, Willerson JT, Casscells SW. Leukocyte count and coronary heart disease: implications for risk assessment. J Am Coll Cardiol 2004; 44(10):1945-56.

5. Iqbal N, Wentworth B, Choudhary R, Landa Ade L, Kipper B, Fard A, Maisel AS. Cardiac biomarkers: new tools for heart failure management. Cardiovasc Diagn Ther. 2012; 2(2):147-64.

6. Cohn JN, Ferrari R, Sharpe N. Cardiac remodeling: concepts and clinical implications. A consensus paper from an international forum on cardiac remodeling. J Am Coll Cardiol. 2000; 35:569-582.

7. Savoye Ch, Equine O, Tricot O, et al. Left Ventricular Remodeling After Anterior Wall Acute Myocardial Infarction in Modern Clinical Practice (from the REmodelage VEntriculaire [REVE] Study Group). Am J Cardiol 2006; 98:1144 - 1149.

8. Sinnaeve, P.R.; Donahue, M.P.; Grass, P.; Seo, D.; Vonderscher, J.; Chibout, S.D.; Kraus, W.E.; Sketch, M.; Nelson, C.; Ginsburg, G.S.; Goldschmidt-Clermont, P.J.; Granger, C.B. Gene expression patterns in peripheral blood correlate with the extent of coronary artery disease. PLoS One. 2009, 4(9), e7037.

9. Joehanes R, Johnson AD, Barb JJ, Raghavachari N, Liu P, Woodhouse KA,O'Donnell CJ, Munson PJ, Levy D. Gene expression analysis of whole blood,peripheral blood mononuclear cells, and lymphoblastoid cell lines from the Framingham Heart Study. Physiol Genomics. 2012; 44(1):59-75.

10. Barton PJ, Birks EJ, Felkin LE, Cullen ME, Koban MU, Yacoub MH. Increased expression of extracellular matrix regulators TIMP1 and MMP1 in deteriorating heart failure. J Heart Lung Transplant. 2003; 22(7):738-44.

11. Polyakova V, Loeffler I, Hein S, Miyagawa S, Piotrowska I, Dammer S, RisteliJ, Schaper J, Kostin S. Fibrosis in endstage human heart failure: severe changes in collagen metabolism and MMP/TIMP profiles. Int J Cardiol. 2011; 151(1):18-33.

12. Ahmed SH, Clark LL, Pennington WR, Webb CS, Bonnema DD, Leonardi AH, McClure CD, Spinale FG, Zile MR. Matrix metalloproteinases/tissue inhibitors of metalloproteinases: relationship between changes in proteolytic determinants of matrix composition and structural, functional, and clinical manifestations of hypertensive heart disease. Circulation. 2006; 113(17):2089-96.

13. Vo TK, de Saint-Hubert M, Morrhaye G, Godard P, Geenen V, Martens HJ, Debacq-Chainiaux F, Swine C, Toussaint O. Transcriptomic biomarkers of the response of hospitalized geriatric patients admitted with heart failure. Comparison to hospitalized geriatric patients with infectious diseases or hip fracture. Mech Ageing Dev. 2011; 132(3):131-9.

14. Yang D, Chen Q, Rosenberg HF, Rybak SM, Newton DL, Wang ZY, Fu Q, Tchernev VT, Wang M, Schweitzer B, Kingsmore SF, Patel DD, Oppenheim JJ, Howard OM. Human ribonuclease A superfamily members, eosinophil-derived neurotoxin and pancreatic ribonuclease, induce dendritic cell maturation and activation. J Immunol. 2004; 173(10):6134-42.

15. Shaulian E., Karin M.: AP-1 as a regulator of cell life and death. Nat. Cell Biol., 2002; 4: E131-E136.

16. Katz S, Heinrich R, Aronheim A. The AP-1 repressor, JDP2, is a bona fide substrate for the c-Jun N-terminal kinase. FEBS Lett. 2001; 506(3):196-200.

17. Sugden PH. Signalling pathways in cardiac myocyte hypertrophy. Ann Med. 2001; 33(9):611-22.

18. Hill C, Würfel A, Heger J, Meyering B, Schlüter KD, Weber M, Ferdinandy P, Aronheim A, Schulz R, Euler G. Inhibition of AP-1 signaling by JDP2 overexpression protects cardiomyocytes against hypertrophy and apoptosis induction. Cardiovasc Res. 2013; 99(1):121-8.

19. Surma M, Wei L, Shi J. Rho kinase as a therapeutic target in cardiovascular disease. Future Cardiol. 2011; 7(5):657-71.

20. Young KG, Copeland JW. Formins in cell signaling. Biochim Biophys Acta. 2010; 1803(2):183-90.

**Claims**

1. A method for diagnosis *in vitro* of the risk of the patient post-infarction heart failure development comprising:

   - determining a gene expression profile of all the gene transcripts from the group consisting of: FMN1, RNASE1 and JDP2 in leukocytes in a biological sample collected from a patient after acute myocardial infarction, and
   - comparing said expression profile to the expression level in non-heart failure patients,
   - wherein an increased expression of all gene transcripts is indicative of heart failure development in the subject.

2. The method of claim 1, wherein the biological sample is selected from blood, blood cells, serum, plasma or preferably peripheral blood mononuclear cells.

3. The method of claim 2, wherein the gene transcripts are determined by PCR, preferably quantitative RT-PCR.

4. A method for determining predisposition to heart failure development for post-infarction patients by determining markers in biological material samples collected from a patient, wherein said method comprises determining increased levels of expression of all the markers from the transcript group consisting of: *FMN1, RNASE1 and JDP2,* when comparing said expression profile to the expression level in non-heart failure patients.

5. A method for optimization of treating regime for cardiac patients based on a result of determination of markers in samples of biological material collected from a patient, wherein said method comprises determining increased levels of expression of all the markers from the transcript group consisting of: *FMN1, RNASE1* and *JDP2* and wherein increased level of expression *FMN1, RNASE1* and *JDP2,* as compared to the expression level in non-heart failure patients, indicates predisposition to heart failure development after myocardial infarction.

**Patentansprüche**

1. Ein Verfahren zur *in vitro* Diagnose des Risikos eines Patienten nach einem Infarkt Herzinsuffizienz zu entwickeln, beinhaltet:

   - Bestimmen eines Genexpressionsprofils aller Gentranskripte aus der Gruppebestehend aus: FMN1, RNASE1 und JDP2 in Leukozyten in einer biologischen Probe, die einem Patienten nach einem akuten Herzinfarkt entnommen wurde und
   - Vergleichen des Expressionsprofils mit dem Expressionsniveau in Patienten ohne Herzinsuffizienz,
   - wobei eine erhöhte Expression aller Gentranskripte auf die Entwicklung einer Herzinsuffizienz bei dem Patienten hinweist.

2. Das Verfahren nach Anspruch 1, wobei die biologische Probe ausgewählt ist aus Blut, Blutzellen, Serum, Plasma oder vorzugsweise mononukleären Zellen des peripheren Blutes.

3. Das Verfahren nach Anspruch 2, wobei die Gentranskripte durch PCR, vorzugsweise quantitative RT-PCR, bestimmt werden.

**4.** Ein Verfahren zur Bestimmung von Prädisposition für die Entwicklung einer Herzinsuffizienz in Patienten nach Infarkt durch Bestimmen von Markern in biologischen Probenmaterial, das von einem Patienten entnommen wurde, wobei das Verfahren die Bestimmung erhöhter Expressionsniveaus aller Marker aus der Transkriptgruppe umfasst, bestehend aus: *FMN1, RNASE1* und *JDP2,* wobei das Expressionsprofil mit dem Expressionsniveau bei Patienten ohne Herzinsuffizienz verglichen wird.

**5.** Ein Verfahren zur Optimierung des Behandlungsregimes für Herzpatienten basierend auf einem Ergebnis von der Bestimmung von Markern in biologischem Probenmaterial, das einem Patienten entnommen wurde, wobei das Verfahren das Bestimmen erhöhter Expressionsniveaus aller Marker aus der Transkriptgruppe umfasst, bestehend aus: *FMN1, RNASE1* und *JDP2* und wobei ein erhöhter Expressionsgrad *FMN1, RNASE1* und *JDP2* im Vergleich zum Expressionsgrad bei Patienten ohne Herzinsuffizienz eine Prädisposition für die Herzinsuffizienzentwicklung nach einem Herzinfarkt anzeigt.

**Revendications**

**1.** Une méthode pour le diagnostic *in vitro* du risque du développement de l'insuffisance cardiaque du patient après un infarctus comprenant :

- la détermination d'un profil d'expression génique de tous les transcrits de gènes du groupe constitué de *FMN1, RNASE1 et JDP2* dans les leucocytes dans un échantillon biologique prélevé chez un patient après un infarctus aigu du myocarde et
- la comparaison dudit profil d'expression avec le niveau d'expression chez des patients ne souffrant pas d'insuffisance cardiaque,

dans laquelle une expression accrue de tous les transcrits de gènes indique un développement de l'insuffisance cardiaque chez le sujet.

**2.** La méthode selon la revendication 1, dans laquelle l'échantillon biologique est choisi parmi le sang, les cellules sanguines, le sérum, le plasma ou de préférence les cellules mononucléaires du sang périphérique.

**3.** La méthode selon la revendication 2, dans laquelle les transcrits de gènes sont déterminés par PCR, de préférence par RT-PCR quantitative.

**4.** Une méthode de détermination d'une prédisposition au développement de l'insuffisance cardiaque chez des patients après un infarctus en déterminant des marqueurs dans des échantillons de matériel biologique prélevés chez un patient, dans laquelle ladite méthode comprend la détermination de niveaux d'expression accrus de tous les marqueurs du groupe de transcription constitué de *FMN1, RNASE1 et JDP2,* en comparant ledit profil d'expression au niveau d'expression chez des patients ne souffrant pas d'insuffisance cardiaque.

**5.** Une méthode d'optimisation du régime de traitement pour les patients cardiaques basée sur le résultat de la détermination de marqueurs en matériel biologique collecté chez un patient, dans laquelle ladite méthode comprend la détermination de niveaux accrus d'expression de tous les marqueurs du groupe de transcription constitué de *FMN1, RNASE1 et JDP2,* et dans laquelle un niveau d'expression accru par rapport à *FMN1, RNASE1 et JDP2,* par rapport au niveau d'expression chez les patients ne souffrant pas d'insuffisance cardiaque, indique une prédisposition au développement de l'insuffisance cardiaque après un infarctus du myocarde.

**Fig. 1**

**Fig. 2**

PCA Mapping (43.8%)

**Fig3**

**Hierarchical Clustering**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110294683 A

**Non-patent literature cited in the description**

- **ANWARUDDIN S ; ASKARI AT ; TOPOL EJ.** Redefining risk in acute coronary syndromes using molecular medicine. *J Am Coll Cardiol.,* 2007, vol. 49 (3), 279-89
- **HORNE BD ; CARLQUIST JF ; MUHLESTEIN JB ; NICHOLAS ZP ; ANDERSON JL.** Intermountain Heart Collaborative Study Group. Associations with myocardial infarction of six polymorphisms selected from a three-stage genome-wide association study. *J. Am Heart,* 2007, vol. 154 (5), 969-75
- **MADJID M ; AWAN I ; WILLERSON JT ; CASS-CELLS SW.** Leukocyte count and coronary heart disease: implications for risk assessment. *J Am Coll Cardiol,* 2004, vol. 44 (10), 1945-56
- **IQBAL N ; WENTWORTH B ; CHOUDHARY R ; LANDA ADE L ; KIPPER B ; FARD A ; MAISEL AS.** Cardiac biomarkers: new tools for heart failure management. *Cardiovasc Diagn Ther.,* 2012, vol. 2 (2), 147-64
- **COHN JN ; FERRARI R ; SHARPE N.** Cardiac remodeling: concepts and clinical implications. A consensus paper from an international forum on cardiac remodeling. *J Am Coll Cardiol.,* 2000, vol. 35, 569-582
- **SAVOYE CH ; EQUINE O ; TRICOT O et al.** Left Ventricular Remodeling After Anterior Wall Acute Myocardial Infarction in Modern Clinical Practice (from the REmodelage VEntriculaire [REVE] Study Group). *Am J Cardiol,* 2006, vol. 98, 1144-1149
- **SINNAEVE, P.R. ; DONAHUE, M.P. ; GRASS, P. ; SEO, D. ; VONDERSCHER, J. ; CHIBOUT, S.D. ; KRAUS, W.E. ; SKETCH, M. ; NELSON, C. ; GINSBURG, G.S.** Gene expression patterns in peripheral blood correlate with the extent of coronary artery disease. *PLoS One.,* 2009, vol. 4 (9), e7037
- **JOEHANES R ; JOHNSON AD ; BARB JJ ; RAGHAVACHARI N ; LIU P ; WOODHOUSE KA ; O'DONNELL CJ ; MUNSON PJ ; LEVY D.** Gene expression analysis of whole blood, peripheral blood mononuclear cells, and lymphoblastoid cell lines from the Framingham Heart Study. *Physiol Genomics.,* 2012, vol. 44 (1), 59-75

- **BARTON PJ ; BIRKS EJ ; FELKIN LE ; CULLEN ME ; KOBAN MU ; YACOUB MH.** Increased expression of extracellular matrix regulators TIMP1 and MMP1 in deteriorating heart failure. *J Heart Lung Transplant.,* 2003, vol. 22 (7), 738-44
- **POLYAKOVA V ; LOEFFLER I ; HEIN S ; MIYAGAWA S ; PIOTROWSKA I ; DAMMER S ; RISTELI J ; SCHAPER J ; KOSTIN S.** Fibrosis in endstage human heart failure: severe changes in collagen metabolism and MMP/TIMP profiles. *Int J Cardiol.,* 2011, vol. 151 (1), 18-33
- **AHMED SH ; CLARK LL ; PENNINGTON WR ; WEBB CS ; BONNEMA DD ; LEONARDI AH ; MCCLURE CD ; SPINALE FG ; ZILE MR.** Matrix metalloproteinases/tissue inhibitors of metalloproteinases: relationship between changes in proteolytic determinants of matrix composition and structural, functional, and clinical manifestations of hypertensive heart disease. *Circulation,* 2006, vol. 113 (17), 2089-96
- **VO TK ; DE SAINT-HUBERT M ; MORRHAYE G ; GODARD P ; GEENEN V ; MARTENS HJ ; DE-BACQ-CHAINIAUX F ; SWINE C ; TOUSSAINT O.** Transcriptomic biomarkers of the response of hospitalized geriatric patients admitted with heart failure. Comparison to hospitalized geriatric patients with infectious diseases or hip fracture. *Mech Ageing Dev.,* 2011, vol. 132 (3), 131-9
- **YANG D ; CHEN Q ; ROSENBERG HF ; RYBAK SM ; NEWTON DL ; WANG ZY ; FU Q ; TCHERNEV VT ; WANG M ; SCHWEITZER B.** Human ribonuclease A superfamily members, eosinophil-derived neurotoxin and pancreatic ribonuclease, induce dendritic cell maturation and activation. *J Immunol.,* 2004, vol. 173 (10), 6134-42
- **SHAULIAN E. ; KARIN M.** AP-1 as a regulator of cell life and death. *Nat. Cell Biol.,* 2002, vol. 4, E131-E136
- **KATZ S ; HEINRICH R ; ARONHEIM A.** The AP-1 repressor, JDP2, is a bona fide substrate for the c-Jun N-terminal kinase. *FEBS Lett.,* 2001, vol. 506 (3), 196-200
- **SUGDEN PH.** Signalling pathways in cardiac myocyte hypertrophy. *Ann Med.,* 2001, vol. 33 (9), 611-22

- **HILL C ; WÜRFEL A ; HEGER J ; MEYERING B ; SCHLÜTER KD ; WEBER M ; FERDINANDY P ; ARONHEIM A ; SCHULZ R ; EULER G.** Inhibition of AP-1 signaling by JDP2 overexpression protects cardiomyocytes against hypertrophy and apoptosis induction. *Cardiovasc Res.,* 2013, vol. 99 (1), 121-8

- **SURMA M ; WEI L ; SHI J.** Rho kinase as a therapeutic target in cardiovascular disease. *Future Cardiol.,* 2011, vol. 7 (5), 657-71
- **YOUNG KG ; COPELAND JW.** Formins in cell signaling. *Biochim Biophys Acta,* 2010, vol. 1803 (2), 183-90